# EUROPEAN PATENT SPECIFICATION

(11) **EP 1 309 674 B1**
(45) Date of publication and mention of the grant of the patent: **13.06.2007**
(21) Application number: 01960183.0
(22) Date of filing: 19.07.2001
(51) Int. Cl.: C12N 9/16, C07K 14/38, C12P 41/00, C07B 57/00, C12P 7/42, C07C 51/09

(54) **STEREOSELECTIVE ESTERASE FROM ASPERGILLUS ORYZAE**
STEREOSELEKTIVE ESTERASE AUS ASPERGILLUS ORYZAE
ESTERASE STEREOSELECTIVE ISOLEE D'ASPERGILLUS ORYZAE

(30) Priority: 07.08.2000 WO PCT/DK00/00440; 07.08.2000 WO PCT/DK00/00439; 17.01.2001 DK 200100088
(43) Date of publication of application: 14.05.2003
(73) Proprietor: Novozymes A/S, 2880 Bagsvaerd (DK)
(72) Inventor: ØSTERGAARD, Peter, Rahbek, DK-2830 Virum (DK); HJORT, Carsten, M., DK-2765 Sm rum (DK); DEUSSEN, Heinz-Josef, DK-2860 S borg (DK); ZUNDEL, Magali, DK-2860 S borg (DK); EBDRUP, Soren, 4000 Roskilde (DK); CHRISTENSEN, Søren, DK-4040 Jyllinge (DK); PATKAR, Shamkant, Anant, DK-2880 Lyngby (DK)
(74) Representative: Knudsen, Sten Lottrup
(86) International application number: PCT/DK2001/000508
(87) International publication number: WO 2002/012472

(56) References cited:
- US-A- 5 665 584
- DATABASE BIOSIS [Online] BIOSCIENCES INFORMATION SERVICE, PHILADELPHIA, PA, US; PREV199800171518, volume 27, no. 1, TENKANEN MAIJA: "Action of trichoderma reesei and aspergillus oryzae esterases in the deacetylation of hemicelluloses" retrieved from ISSN 0885-4513 XP002902058
- PATENT ABSTRACTS OF JAPAN vol. 1995, no. 11, 26 December 1995 (1995-12-26) & JP 07 206756 A (NAGASE & CO LTD), 8 August 1995 (1995-08-08)

## Description

### FIELD OF THE INVENTION

The present invention relates to an esterase, to methods of using and producing it, and to a nucleic acid sequence encoding it The esterase is capable of stereoselective hydrolysis of chiral esters and of hydrolyzing ferulic acid esters.

### BACKGROUND OF THE INVENTION

It is known to prepare chiral esters of high optical purities by asymmetric hydrolysis with enzymes. Thus, US 4587462 discloses asymmetric hydrolysis of lower alkyl esters of naproxen with a microbial enzyme, particularly a specific splitting esterase from *Aspergillus oryzae* DSM 2808 (ATCC 11492). US 5155028 discloses enzymatic stereoselective ester cleavage using lipases, esterases or proteases, e.g. lipases or esterases from *Aspergillus* or proteases from *Aspergillus oryzae.* JP 7-206756 A discloses use of an enzyme to prepare optically active compounds. The enzyme may be a protease or esterase produced by *Aspergillus,* e.g. from *A. oryzae.*

Enzymes with ferulic acid esterase activity are known, e.g., from *Aspergillus oryzae.* M. Tenkanen; Biotechnology and Applied Biochemistry, 27 (1), 19-24 (1998)); M. Tenkanen et al., J. Biotechnol., 18 (1-2), 69-84 (1991).

US 5516679 discloses a penicillin V amidohydrolase from *Fusarium oxysporum.*

### SUMMARY OF THE INVENTION

The inventors have isolated an esterase from *Aspergillus oryzae* which is capable of stereoselective hydrolysis of chiral esters and also has arylesterase activity (EC 3.1.1.2) and feruloyl esterase activity (EC 3.1.1.73). The novel esterase has only a limited homology to known amino acid sequences. The inventors also isolated a gene encoding the novel esterase and cloned it into an *E. coli* strain.

Accordingly, the invention provides an esterase which is capable of stereoselective hydrolysis of substituted esters of 3-phenyl-propanoic acid. The esterase may be a polypeptide having an amino acid sequence as the mature peptide shown in SEQ ID NO: 2.

Further, the esterase of the invention may be a polypeptide encoded by the esterase encoding part of the DNA sequence cloned into a plasmid present in *Escherichia coli* deposit number DSM 13977.

The esterase may also be an analogue of the polypeptide defined above which:
i) has at least 50 % identity with said polypeptide,
ii) is immunologically reactive with an antibody raised against said polypeptide in purified form,
iii) is an allelic variant of said polypeptide,

Finally, the esterase of the invention may be a polypeptide which is encoded by a nucleic acid sequence which hybridizes at 60°C, 2 x SSC, 0.5 % SDS with the complementary strand of nucleotides 572-911 and 971-2208 of SEQ ID NO: 1 or a subsequence thereof of at least 100 nucleotides.

The nucleic acid sequence of the invention may comprise a nucleic acid sequence which encodes the esterase described above, or it may encode an esterase and comprise:
a) the esterase encoding part of the DNA sequence cloned into a plasmid present in *Escherichia coli* DSM 13977,
b) nucleotides 629-911 and 971-2208 of SEQ, ID NO: 1 (encoding the mature polypeptide), or
c) an analogue of the DNA sequence defined in a) or b) which encodes a esterase which is capable of stereoselective hydrolysis of substituted esters of 3-phenyl-propanoic acid and
i) has at least 60 % identity with said DNA sequence, or
ii) hybridizes at 60°C, 2 x SSC, 0.5 % SDS with the complementary sequence of said DNA sequence.

Other aspects of the invention provide a recombinant expression vector comprising the DNA sequence, and a cell transformed with the DNA sequence or the recombinant expression vector.

A comparison with full-length prior-art sequences shows the closest known sequence is a penicillin V amidohydrolase from *Fusarium oxysporum* (US 5516679). The mature amino acid sequence of the invention has 48 % identity to the known sequence, and the corresponding DNA sequences have 54 % identity.

### BRIEF DESCRIPTION OF THE DRAWING

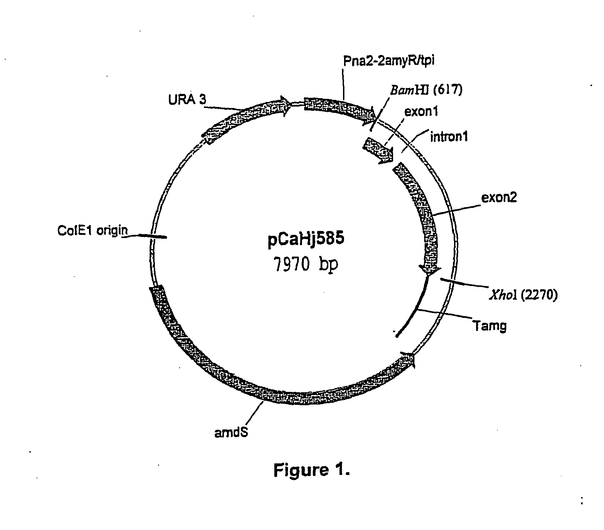

Figure 1 shows a restriction map of the esterase expression plasmid pCaHj585.

### DETAILED DESCRIPTION OF THE INVENTION

### Genomic DNA source

The esterase of the invention may be derived from strains of *Aspergillus,* particularly strains of *A. oryzae,* using probes designed on the basis of the DNA sequence in this specification.

A strain of *Escherichia coli* containing a gene encoding the esterase was deposited by the inventors under the terms of the Budapest Treaty with the DSMZ - Deutsche Sammlung von Microorganismen und Zellkulturen GmbH, Mascheroder Weg 1b, D-38124 Braunschweig DE on 11 January 2001 under accession number DSM 13977

### Recombinant expression vector

The expression vector of the invention typically includes control sequences encoding a promoter, operator, ribosome binding site, translation initiation signal, and, optionally, a selectable marker, a transcription terminator, a repressor gene or various activator genes. The vector may be an autonomously replicating vector, or it may be integrated into the host cell genome.

### Production by cultivation of transformant

The esterase of the invention may be produced by transforming a suitable host cell with a DNA sequence encoding the esterase, cultivating the transformed organism under conditions permitting the production of the enzyme, and recovering the enzyme from the culture.

The host organism may be a eukaryotic cell, in particular a fungal cell, such as a yeast cell or a filamentous fungal cell, such as a strain of *Aspergillus, Fusarium, Trichoderma* or *Saccharomyces,* particularly *A. niger, A. oryzae, F. graminearum, F. sambucinum, F. cerealis* or *S. cerevisiae.* The production of the esterase in such host organisms may be done by the general methods described in EP 238,023 (Novo Nordisk), WO 96/00787 (Novo Nordisk) or EP 244,234 (Alko).

### Properties and uses of esterase

The esterase of the invention is capable of stereoselective hydrolysis of esters including substituted esters of 3-phenyl-propanoic acids. It does not hydrolyse naproxen ethyl ester.

The esterase is useful for the preparation of optically enriched esters or acids, e.g. substituted esters of 3-phenyl-propanoic acids and substituted 3-phenyl-propanoic acids for pharmaceutical use.

The esterase also has arylesterase activity (EC 3.1.1.2) and feruloyl esterase activity (EC 3.1.1.73) and is useful in hydrolyzing feruloyl esters into ferulic acid and alcohol. It is useful for the release of ferulic acid bound to hemicellulose in the degradation of plant material and plant cell walls, e.g. as described in GB 2301103 and WO 200014234. It may also be used for the production of vanillic acid in analogy with US 5955137.

### Hybridization

The hybridization is used to indicate that a given DNA sequence is analogous to a nucleotide probe corresponding to a DNA sequence of the invention. The hybridization conditions are described in detail below.

Suitable conditions for determining hybridization between a nucleotide probe and a homologous DNA or RNA sequence involves presoaking of the filter containing the DNA fragments or RNA to hybridize in 5 x SSC (standard saline citrate) for 10 min, and prehybridization of the filter in a solution of 5 x SSC (Sambrook et al. 1989), 5 x Denhardt's solution (Sambrook et al. 1989), 0.5 % SDS and 100 µg/ml of denatured sonicated salmon sperm DNA (Sambrook et al. 1989), followed by hybridization in the same solution containing a random-primed (Feinberg, A. P. and Vogelstein, B. (1983) Anal. Biochem. 132:6-13), ³²P-dCTP-labeled (specific activity > 1 x 10⁹ cpm/µg) probe for 12 hours at approx. 60°C. The filter is then washed two times for 30 minutes in 2 x SSC, 0.5 % SDS at a temperature of 60 °C, more preferably at least 65°C, even more preferably at least 68°C.

Molecules to which the oligonucleotide probe hybridizes under these conditions are detected using a x-ray film.

### Alignment and homology

The esterase and the nucleotide sequence of the invention preferably have homologies to the disclosed sequences of at least 80 % identity, particularly at least 90 % identity or at least 95 % identity, e.g. at least 98 % identity.

For purposes of the present invention, alignments of sequences and calculation of identityscores were done using a Clustal W (J. D. Thompson et al (1994) NAR 22 (22) p4673 - 4680) alignment, useful for both protein and DNA alignments. The default scoring matrices Blosum62mt2and swgapdnamt are used for protein and DNA alignments respectively. The gap opening penalty is 10 and the gap extension penalty: 0,1 for proteins. - The gap opening penalty is 15 and the gap extension penalty is 6,66 for DNA.. The alignments were done using the computer programme allignX which is a component of the Vector NTI Suite 6.0 package from Informax, Inc (www.informax.com))

### EXAMPLES

### Example 1: Preparation of a crude esterase preparation from Aspergillus oryzae:

*Aspergillus oryzae* IFO4177 was fermented using a fed-batch process with maltose/maltodextrin or glucose as the main carbon source. The batch medium contained: maltose/maltodextrin, ammonium sulphate, potassium-dihydrogenphosphate, yeast extract, beech xylan, MgSO4,7H2O, citric acid, potassium sulphate, trace metal solution and an antifoam agent. All these components were used in concentrations all being within the range of 1-18 g/L final medium. The medium pH was kept at 4.5 throughout the fermentation. The feed consisted of maltose/maltodextrin or glucose in the range of 280 g/L. 6.5 kg of batch medium was inoculated with 500 mL of seed culture. After 15-25 hours of batch fermentation the addition of feed was initiated using a feed addition rate of 15-25 g of feed per hour. This fed-batch state was continued for 100-160 hour of fermentation. Dissolved oxygen above 50% saturation was maintained by means of closed-loop control of the agitation rate. Aeration was kept at 1 volume air per volume batch medium per hour. A headspace pressure of 0.5 bar overpressure was maintained throughout the entire fermentation. After harvest of the broth, both biomass and un-dissolvdd matter was removed in a filtration step. The supernatant was concentrated by removal of water using ultrafiltration, evaporation or freeze drying.

### Example 2: Preparation of (2S)-2-Ethoxy-3-(4-hydroxyphenyl)propanoic acid and Ethyl (2R)-2-Ethoxy-3-(4-hydroxyphenyl)propanoate

Ethyl (2RS) (+/-) 2-ethoxy-3-(4-hydroxyphenyl)propanoate (0.5 g) was shaken with 60 mg of the lyophilised esterase preparation from *Aspergillus oryzae* in 1 ml 1 M phosphate buffer (pH=7) with organic co-solvents (according to the table below) at 27°C. The reaction mixture was poured into 20 ml MeOH after 4h to stop the enzymatic reactions followed by analysis by chiral capillary electrophoresis to determine the enantiomeric excess (ee) as follows:

HP 3D Capillary Electrophoresis and 80.5/72.0 cm, 50 mm HP bubble capillary were used. The electrolyte was HS-β-CD (Regis) (2%w/v) and TM-β-CD (Sigma) (2%w/v) in 25 mM borate buffer pH 9.3 (HP). The reaction mixture was diluted approximately 25 times in borate buffer 5 mM, pH 9.3 (or final concentration ca. 0.025mg/ml - 0.1 mg/ml) and injected (50 mbar in 4.0 seconds). The applied voltage was 30kV.

| Co-solvent | Product_{acid} (%) | (ee)_{acid} (%) |
|---|---|---|
| Acetone/0.1 ml | 37 | 93 |
| Acetone / 0.3 ml | 31 | 94 |
| THF/0.1 ml | 36 | 94 |
| THF/0.2 ml | 31 | 93 |
| THF/0.3 ml | 21 | 91 |
| 2-Propanol/0.1, ml | 36 | 97 |
| 2-Propanol / 0.3 ml | 27 | 93 |
| Ethanol/ 0.1 ml | 35 | 96 |
| Ethanol / 0.2 ml | 32 | 96 |
| Ethanol / 0.3 ml | 22 | 93 |

### Example 3: Preparation of (2S)-2-Ethoxy-3-(4-hydroxyphenyl)propanoic acid and Ethyl (2R)-2-Ethoxy-3-(4-hydroxyphenyl)propanoate

Ethyl (2*R*/*S*) (+/-) 2-ethoxy-3-(4-hydroxyphenyl)propanoate (5 g) was added to an aqueous 0.1 M phosphate buffer pH 7 (10 ml). 100 mg of the lyophilised esterase preparation from *Aspergillus oryzae* was added and the mixture was stirred for 18 hours at room temperature. During that time, the pH of the reaction mixture was kept constant at pH=6-8 by addition of NaOH. Most of the water was evaporated *in vacuo.* Methanol was added to the remaining slurry in order to stop the hydrolysis. The precipitate, which formed was filtered off and the methanol was evaporated *in vacuo.* The remaining oil was dissolved in water followed by extraction of unreacted ester with tert-butyl methyl ether (TBME) (eeₑₛₜₑᵣ = 87%, determined as in Example 2). The water phase was acidified to pH = 3 and the acid extracted with TBME. After drying over Na₂SO₄ and evaporation of the TBME, 1.8g (2S)-2-Ethoxy-3-(4-hydroxyphenyl)propanoic acid was obtained as an oil, which crystallized on standing (m.p. = 105°C, ee_{acid} = >99 %, determined as in Example 2).

### Example 4: Purification of the esterase.

### Fermentation of Aspergillus oryzae IFO4177

Fed batch fermentation of a derivative of *Aspergillus oryzae* IFO4177 was performed in a medium comprising maltodextrin as a carbon source, urea as a nitrogen source and yeast extract. The fed batch fermentation was performed by inoculating a shake flask culture of A, *oryzae* into a medium comprising 3.5% of the carbon source and 0.5% of the nitrogen source. After 24 hours of cultivation at pH 5.0 and 34°C the continuous supply of additional carbon and nitrogen sources were initiated. The carbon source was kept as the limiting factor and it was secured that oxygen is present in excess. The fed batch cultivation was continued for 4 days, after which the enzyme was recovered by centrifugation, ultrafiltration, filtration, germ filtration and spray drying.

### Assay for ester hydrolysis:

The assay is an pH indicator based assay, where the decrease in pH is measured with p-Nitrophenol, when the enzyme cleaves the ester Ethyl (2RS) (+/-) 2-ethoxy-3-(4-hydroxyphenyl)propanoate. The buffer capacity of the enzyme sample in question has to be low, as a high buffer capacity in the sample will suppress the pH drop.

50µl enzyme (diluted in 5mM BES (Sigma B-6420), pH 7.1) was mixed with 100µl Assay solution (a mixture of 400µl Ethyl (2RS) (+/-) 2-ethoxy-3-(4-hydroxyphenyl)propanoate (55mM in acetonitril), 2000µl 5mM p-Nitrophenol in 5mM BES, pH 7.1 and 7600µl 5mM BES, pH 7.1). After a 5 minutes lag period, the decrease in OD₄₀₅ the next 10 minutes was monitored as a measurement of the enzyme activity. If the slope of the monitored curve deviated from linearity, the assay was repeated with a higher enzyme dilution.

### Purification of the esterase

24 g of a spray dried Aspergillus oryzae IFO4177 supernatant was dissolved in 375ml 5mM CH₃COOH/NaOH, pH 4.0 and the pH was adjusted to pH 4.0. The solution had a 1 mS/cm conductivity. The solution was applied to a 100ml S-sepharose FF column (Amersham Pharmacia Biotech) equilibrated in 25mM CH₃COOH/NaOH, pH 4.0. After washing the column with the same buffer, the column was eluted with a linear NaCl gradient from 0 to 0.5M over 5 column volumes. Fractions (10ml) from the column were analyzed for esterase activity and fractions 42 - 46 were pooled. The 42-46 pool was dialysed over night against 10mM KH₂PO₄/NaOH, pH 7.0 in dialysis tubing and then applied to a 40ml Q-sepharose FF column (Amersham Pharmacia Biotech) column equilibrated in 20mM KH₂PO₄/NaOH, pH 7.0. After washing the column with the same buffer, the column was eluted with a linear NaCl gradient from 0 to 0.25M over 5 column volumes. Fractions from the column were analysed for activity. The activity was found in the unretained fraction. The pH of the unretained fraction was adjusted to pH 8.0 and applied to the same 40ml Q-sepharose FF column, but this time equilibrated in 20mM HEPES/NaOH, pH 8.0. After washing the column with the HEPES buffer, the column was eluted with a linear NaCl gradient from 0 to 0.25M over 5 column volumes. Fractions (4ml) from the column were analysed for activity and fractions 18-22 were pooled. The 18-22 pool was diluted 10 times with deionised water and applied to an 8ml SOURCE Q column (Amersham Pharmacia Biotech) equilibrated in 20mM HEPES/NaOH, pH 8.0. After washing the column with the same buffer, the column was eluted with a linear NaCl gradient from 0 to 150mM over 30 column volumes. Fraction (3ml) 28 and 29 was pooled and applied to a 300ml Superdex75 column (Amersham Pharmacia Biotech) equilibrated in 20mM HEPES/NaOH, 200mM NaCl, pH 8.0. The Superdex75 column was eluted with the same buffer and fractions (5ml) were analyzed for activity. The enzyme activity peaked in fraction 6 and 7. Fraction 5, 6, 7 and 8 were concentrated to approx. 70µl in 10 kDa cut-off polysulfone Ultrafuge units (ultrafiltration by centrifugation). 10µl of each fraction were applied to a SDS-PAGE gel, and it was seen that the intensity of a ∼70 kDa band followed the activity. Fraction 6 and 7 were used for cleavage and Edman protein sequencing.

### Example 5: Cleavage of the esterase into fragments and sequencing of the fragments.

### Reduction and alkylation

75µl of the purified enzyme sample was mixed with 75µl SDS PAGE sample buffer with DTT and incubated at 37°C for 20 min. The sample was heated to 95°C for 3 min, cooled and subsequently 20µl 1 M iodoacetamide (in 0.5M Tris pH 9.2) was added. Incubation for 20 min at room temperature.

### In-Gel digestion

Nine lanes in a Novex SDS-PAGE gel were loaded with the sample (all). After running the gel it was stained according to standard procedures from Novex Pieces of the gel holding the -70 kDa band was subsequently cut out and minced with a blade. The gel pieces was washed 2X in 0.5M Tris pH 9.2/Acetonitrile (ACN) (1:1) for 45 min at 37°C. The gel pieces were treated with 100% ACN for 10 min to introduce shrinking of the pieces. The ACN was removed and the pieces dried in a speed-Vac. 200µl 0.1M (NH₄)HCO₃ was added and incubated for 15 min. The (NH₄)HCO₃ was removed and 100µl ACN added. Again incubation for 10 min followed by removal of ACN. and drying in a speed-vac. The cycle with alternating (NH₄)HCO₃ and ACN addition/removal was repeated 2X. After the last drying step 25µl 0.1µg/µl Acromobacter lysyl endopeptidase in 0.1 M Tris pH 9.2, 10% ACN was added. Incubation for 20 min. Then 350µl 0.1M Tris pH 9.2, 10% ACN was added. Incubation was continued over night at 37°C.

Then 40µl 10% Trifluoroacetic acid (TFA) was added and after a 10 min incubation, the supernatant was removed (saved for control). Extraction of peptides was done 2 X by adding 200µl 0.1% TFA, 60% ACN to the gel pieces and incubate for 45 min at 37°C. All extracts were collected (65µl+200µl+200 µl) and concentrated in the speed-vac to 50 µl. 50 µl 0.1 % TFA was added and the sample re-dried to 50µl.

### Separation of peptides

The sample was run on RP-HPLC on a 2x50 mm Vydac C-18 column using a TFA/ACN solvent system (gradient from 0% to 64% ACN in 0.1% TFA over 31 min, 150 µl/min, detection at 214nm). Controls with blank gel pieces were run in parallel. Selected peptides from the separation were subjected to sequence analysis by Edman degradation.

### Peptide sequences

Sequence analysis of the peptides showed that three sequences was obtained. The determined sequences were denoted 161299Afr15 (SEQ ID NO: 3), 161299Afr17 (SEQ ID NO: 4) and 161299Afr23 (SEQ ID NO: 5).

### Example 6: Cloning of the esterase gene.

### Partial cloning of the esterase gene by PCR.

The three peptide sequences determined in example 5 were all found to show some homology to a Penicillin V amidohydrolase from Fusarium oxysporum (GeneSeqP: W00290). Thus, 161299Afr15 (SEQ ID NO: 3), 161299Afr17 (SEQ ID NO: 4) and 161299Afr23 (SEQ ID NO: 5) could be aligned with amino acids 381-398, 349-366 and 181-201, respectively, of GeneSeqP: W00290.

Based, on this alignment, two PCR primers were designed to PCR amplify a part of the A. *oryzae* esterase gene.

A primer (B2716F09, SEQ ID NO: 6) corresponding to the sense direction protein sequence of peptide 161299Afr23 (amino acids 13-21 of SEQ ID NO: 5) was synthesized.

A primer (B2716F11, SEQ ID NO: 7) corresponding to the antisense direction of the protein sequence of peptide 161299Afr15 (amino acids 4-12 of SEQ ID NO: 3) was synthesized.

These two PCR primers were used for amplification of an esterase gene fragment in the following way:

Genomic DNA was prepared from *Aspergillus oryzae* IFO 4177 as described by Yelton et. al. (M. M. Yelton, J. E. Mamer and W. E. Timberlake (1984) Proc. Natl. Acad. Sci. USA 81,1470-1474).

The Expand PCR system (Roche Molecular Biochemicals, Basel, Switzerland) was used for the amplification following the manufacturers instructions for this and the subsequent PCR amplifications. The magnesium concentration was held at 2.5 mM in all PCR reactions.

The following thermal cycling program was run on an MJ Research PTC 150 thermal cycler:

| | |
|---|---|
| 94°C for 1 min. | 1 cycle |
| 94°C for 10 sec. | |
| Temperature ramping at -0,5°C/sec. | |
| 50°C for 10 sec. | 40 cycles |
| 72°C for 30 sec. | |
| 72°C for 1 min. | 1 cycle. |

A PCR product of approx. 550 bp were detected on a 1 % agarose gel. This fragment was recovered and cloned into the pCR4-TOPO vector following the manufacturers instructions (Invitrogen BV, Groningen, the Netherlands).

A plasmid with an insert of the expected size, pCaHj571, was selected for sequencing and it was sequenced using the following primers: -48 reverse (SEQ ID NO: 8) and -40 universal (SEQ ID NO: 9).

All sequence reactions were made using BigDye^{™} Terminator Cycle Sequencing Kits from the Perkin-Elmer Corporation (USA), and the reactions were run on an ABI 3700 capillary sequencer from the Perkin-Elmer Corporation following the manufacturers instructions.

The sequence encoded a protein sequence homologous to the *F. oxysporum* amidohydrolase and also encoded the peptide sequence 161299Afr17, and it was thus concluded that the amplified fragment is a part of the esterase gene. The sequence of the PCR fragment is shown as SEQ ID NO: 27.

### Genomic cloning of the esterase gene.

A cosmid library of A. oryzae IFO4177 has previously been prepared as described in WO 9801470.

The insert of pCaHj571 was labelled with DIG by using the plasmid as template in a PCR reaction together with the primers B2716F09 and B2716F11 and the PCR DIG probe synthesis kit from Roche Molecular Biochemicals following the manufacturers instructions.

The DIG labeled fragment was used to probe filters of the cosmid library using the recomandations of Roche Molecular Biochemicals, and the hybridization signals were visualized using CSPD detection following the instructions of Roche Molecular Biochemicals and using a LAS1000 plus CDC camera manufactured by Fujifilm.

One cosmid giving a clear hybridization signal was isolated and termed pCaHj577.

A southern blot using genomic DNA of A. oryzae IFO4177 was done using the same probe, hybridization conditions and detection method as for the cosmid isolation. The DNA was digested with the restriction enzymes BamH I, Bgl II, EcoR I, Hind III, Mlu I, Mun I, Sac I, SaL I or Xho I. In addition an Asp 718 digestion was made, and double digestions of Asp 718 and the list of enzymes just indicated was made.

The Southern blot indicated that the 5' end of the esterase gene was present on an approx. 1.4 kb Asp718 fragment. The 3' end of the gene appeared to be present on an approx 2 kb Mun I - EcoR I fragment.

The 5' and the 3' end of the gene was cloned by inverse PCR in the following way:

From the sequence given in SEQ ID NO: 27, the following PCR primers were designed: B2998F06 (SEQ ID NO: 10), B3591G12 (SEQ ID. NO: 11) and B2998F07 (SEQ ID NO: 12).

For the 5' inverse PCR 500 ng of pCaHj577 was digested with Asp718, and the formed fragments were separated on a 1 % agarose gel. Fragments of approx. 1.4 kb were recovered from the gel and dissolved in a total volume of 0.5 ml. Ligation buffer and T4 DNA ligase (Roche Molecular Biochemicals) was added and the solution was incubated at 16°C for approx. 18 hours. The mixture was concentrated by ethanol precipitation and the ligation product was used as template in a PCR reaction using the primers B2998F06 and B3591 G12 and the PCR conditions described in the previous section. A PCR product of approx. 1 kb was detected on a 1 % agarose gel. This fragment was recovered and cloned into the pCR4-TOPO vector. Sequencing of one of the formed plasmids demonstrated the insert to be the 5' end of the esterase. This plasmid was named pCaHj578.

For the 3' inverse PCR 500 ng of pCaHj577 was digested with Mun I and EcoR I, and the formed fragments were separated on a 1% agarose gel. Fragments of approx. 2 kb were recovered from the gel and dissolved in a total volume of 0.5 ml. Ligation and concentration was done as with the 5' end. The ligation product was used as template in a PCR reaction using the primers B2998F06 and B2998F07. A PCR product of approx. 1.1 kb was detected on a 1% agarose gel. This fragment was recovered and cloned into the pCR4-TOPO vector. Sequencing of one of the formed plasmids demonstrated the insert to be the 3' end of the esterase. This plasmid was named pCaHj579.

### Sequencing of the esterase gene.

The esterase gene was sequenced using the plasmids pCaHj571, pCaHj 577, pCaHj578 and pCaHj579 as templates and the following primers:

-48 reverse (SEQ ID NO: 8), -40 universal (SEQ ID NO: 9), B2998F06 (SEQ ID NO: 10), B3591G12 (SEQ ID NO: 11), B2998F07 (SEQ ID NO: 12), B3864E07 (SEQ ID NO: 13), B3864E08 (SEQ. ID NO: 14), B3998D09 (SEQ ID NO: 15), B3998D10 (SEQ ID NO: 16), B3998D11 (SEQ ID NO: 17), and B3591G11 (SEQ ID NO:18).

All sequence reactions were made using BigDye^{™} Terminator Cycle Sequencing Kits from the Perkin-Elmer Corporation (USA), and the reactions were run on an ABI 3700 capillary sequencer from the Perkin-Elmer Corporation following the manufacturers instructions.

The sequence is shown together with the translation as SEQ ID NO: 1. A single intron was predicted by the computer programme NetGene2 (P. G. Koming et. al (1996) Nucl. Acids. Res. 24: 3439-3452).

By analysis of the protein sequence a secretory signal sequence was predicted using the computer programme SignalP (H. Nielsen et al (1997) Protein Eng. 10:1-6).

### Example 7: Expression of the esterase gene.

The Aspergillus expression plasmid pCaHj527 (WO 0070064) consists of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminater (Tamg). Also present on the plasmid is the *Aspergillus* selective marker *amdS* from *Aspergillus nidulans* enabling growth on acetamide as sole nitrogen source and the URA3 marker from *Saccharomyces cerevisiae* enabling growth of the *pyrF* defective *Escherichia coli* strain DB6507 (ATCC 35673). Transformation into *E. coli* DB6507 using the *S. cerevisiae* URA 3 gene as selective marker was done in the following way:

*E. coli* DB6507 was made competent by the method of Mandel and Higa (Mandel, M. and A. Higa (1970) J. Mol. Biol. 45, 154). Transformants were selected on solid M9 medium (Sambrook et. al (1989) Molecular cloning, a laboratory manual, 2. edition, Cold Spring Harbor Laboratory Press) supplemented with 1 g/l casaminoacids, 500 µg/l thiamine and 10 mg/l kanamycin.

PCaHj527 was modified in the following way:

ThePna2/tpi promoter present on pCaHj527 was subjected to site directed mutagenises by a simple PCR approach.

Nucleotide 134 -144 was altered from SEQ ID NO: 19 to SEQ ID NO: 20 using the mutagenic primer 141223 (SEQ ID NO: 21).

Nucleotide 423 - 436 was altered from SEQ ID NO: 22 to SEQ ID NO: 23 using the mutagenic primer 141222 (SEQ ID NO: 24).

The resulting plasmid was termed pMT 2188.

The esterase gene was cloned into pMT2188 in the following way:

The esterase gene was PCR amplified from pCaHj577 using the PCR conditions described in Example 6, except only 20 cycles was used. The primers were the following: B6093H05 (SEQ ID NO: 25) and B6093H03 (SEQ ID NO: 26).

The formed PCR fragment was digested with BamH I and Xho I, and the large fragment formed was ligated to pMT2188 digested with the same enzymes. The ligation mixture was transformed into *E. coli* DB6507. A plasmid from one of the colonies formed was confirmed to have the expected insert by restriction analysis and DNA sequencing. This plasmid was termed pCaHj585. A restriction map of pCaHj585 is shown in figure 1.

PCaHj585 was transformed into Aspergillus oryzae BECh2 (WO 0039322), fermented and recovered as described in WO 95/00636.

### Example 8: Use of esterase for stereoselective hydrolysis of chiral ester

Ethyl (2RS) (+/-) 2-ethoxy-3-(4-hydroxyphenyl)propanoate (0.5 g) was shaken with 60mg of the lyophilised hydrolytic enzyme mixture from *Aspergillus oryzae* in 1 ml 1 M phosphate buffer (pH=7) with organic co-solvents (according to the table below) at 27°C. The reaction mixture was poured into 20 ml MeOH after 4h to stop the enzymatic reactions followed by analysis by the chiral CCE method 2.

| Co-solvent, | Product_{acid} (%) | (ee)_{acid} (%) |
|---|---|---|
| Acetone/0.1 ml | 37 | 93 |
| Acetone/0.3 ml | 31 | 94 |
| THF/0.1 ml | 36 | 94 |
| THF/0.2 ml | 31 | 93 |
| THF/0.3 ml | 21 | 91 |
| 2-Propanol/0.1 ml | 36 | 97 |
| 2-Propanol/0.3 ml | 27 | 93 |
| Ethanol / 0.1 ml | 35 | 96 |
| Ethanol / 0.2 ml | 32 | 96 |
| Ethanol / 0.3 ml | 22 | 93 |

In another experiment, ethyl (2*R*/*S*) (+/-) 2-ethoxy-3-(4-hydroxyphenyl)propanoate (5 g) was added to an aqueous 0.1 M phosphate buffer pH 7 (10 ml). 100mg of the lyophilised hydrolytic enzyme mixture from *Aspergillus oryzae* was added and the mixture was stirred for 18 hours at room temperature. During that time, the pH of the reaction mixture was kept constant at pH=6-8 by addition of NaOH. Most of the water was evaporated *in vacuo.* Methanol was added to the remaining slurry in order to stop the hydrolysis. The precipitate, which formed was filtered off and the methanol was evaporated *in vacuo.* The remaining oil was dissolved in water followed by extraction of unreacted ester with TBME (CCE method 2: eeₑₛₜₑᵣ = 87%). The water phase was acidified to pH = 3 and the acid extracted with TBME. After drying over Na₂SO₄ and evaporation of the TBME, 1.8g (2S)-2-Ethoxy-3-(4-hydroxyphenyl)propanoic acid was obtained as an oil, which crystallized on standing (m.p. = 105°C, CCE method 2: ee_{acid} = >99 %).

## Claims

1. An esterase which is capable of stereoselective hydrolysis of chiral esters and has arylesterase activity and feruloyl esterase activity and which is:
a) a polypeptide encoded by an esterase encoding part of the DNA sequence cloned into a plasmid present in *Escherichia coli* deposit number DSM 13977;
b) a polypeptide having an amino acid sequence as the mature peptide shown in SEQ ID NO: 2;
c) an analogue of the polypeptide defined in (a) or (b) which:
i) has at least 50 % identity with said polypeptide,
ii) is immunologically reactive with an antibody raised against said polypeptide in purified form, or
iii) is an allelic variant of said polypeptide; or
d) a polypeptide which is encoded by a nucleic acid sequence which hybridizes at 60°C, 2 x SSC, 0.5 % SDS with a complementary strand of the nucleic acid sequence shown as nucleotides 629-911 and 971-2208 of SEQ ID NO: 1 or a subsequence thereof having at least 100 nucleotides.

2. The esterase of claim 1 which has been isolated from a strain of *Aspergillus,* preferably *A. oryzae.*

3. A polynucleotide comprising a nucleic acid sequence which encodes the esterase of claim 1 or 2.

4. A polynucleotide which comprises:
a) the esterase encoding part of the DNA sequence cloned into a plasmid present in *Escherichia coli* DSM 13977,
b) the nucleic acid sequence shown as nucleotides 629-911 and 971-2208 of SEQ ID NO: 1,
c) an analogue of the sequence defined in a) or b) which encodes a esterase which is capable of stereoselective hydrolysis of chiral esters and has arylesterase activity and feruloyl esterase activity and has at least 60% identity with said polynucleotide
ii) hybridizes at 60°C, 2 x SSC, 0.5 % SDS with a complementary strand of said DNA sequence or a subsequence thereof having at least 100 nucleotides,
iii) is an allelic variant thereof, or
d) a complementary strand of a), b) or c).

5. A nucleic acid construct comprising the polynucleotide of claim 3 or 4 operably linked to one or more control sequences capable of directing the expression of the esterase in a suitable expression host.

6. A recombinant expression vector comprising the nucleic acid construct of claim 5, a promoter, and transcriptional and translational stop signals.

7. A recombinant host cell transformed with the nucleic acid construct of claim 6.

8. A method for producing an esterase comprising cultivating the host cell of claim 7 under conditions conducive to production of the esterase, and recovering the esterase.

9. The method of the preceding claim wherein the esterase can be derived from the mature peptide of SEQ ID NO: 2 or is an analogue thereof, and the host cell is a transformed strain of *A. oryzae.*

10. A method for stereoselective hydrolysis of a chiral ester, comprising treating the ester with the esterase of claim 1 or 2.

11. A method of producing an optically active carboxylic ester, comprising stereoselectively hydrolyzing a racemic mixture of the ester by treating with the esterase of claim 1 or 2, and recovering the optically active ester.

12. A method of producing an optically active carboxylic acid, comprising stereoselectively hydrolyzing a racemic mixture of an ester of the acid by treating with the esterase of claim 1 or 2, and recovering the optically active acid.

13. A method of hydrolyzing a feruloyl ester into ferulic acid and an alcohol, comprising treating the ester with the esterase of claim 1 or 2.

## Patentansprüche

1. Esterase, welche zur stereoselektiven Hydrolyse chiraler Ester befähigt ist und eine Arylesteraseaktivität und Feruloylesteraseaktivität hat und welche ist:
a) ein Polypeptid, kodiert von einem Esterase-kodierenden Teil einer DNA-Sequenz, die in ein in *Escherichia coli* Hinterlegungsnummer DSM 13977 vorhandenem Plasmid kloniert ist;
b) ein Polypeptid, das als reifes Peptid eine in SEQ ID NO:2 gezeigte Aminosäuresequenz hat;
c) ein Analog des in (a) oder (b) definierten Polypeptids, welches:
i) mindestens 50% Identität mit besagtem Polypeptid aufweist,
ii) mit einem Antikörper, der gegen besagtes Polypeptid in gereinigter Form gerichtet ist, immunologisch reaktiv ist, oder
iii) eine allelische Variante des besagten Polypeptids ist; oder
d) ein Polypeptid, welches von einer Nukleinsäuresequenz kodiert wird, welche bei 60°C, 2xSSC, 0,5% SDS mit einem komplementären Strang der als Nukleotide 629-911 und 971-2208 der SEQ ID NO:1 gezeigten Nukleinsäuresequenz oder einer Untersequenz davon mit mindestens 100 Nukleotiden hybridisiert.

2. Esterase nach Anspruch 1, welche aus einem Stamm von *Aspergillus,* bevorzugt A. *oryzae* isoliert worden ist.

3. Polynukleotid umfassend ein Nukleinsäuresequenz, welche die Esterase nach Anspruch 1 oder 2 kodiert.

4. Polynukleotid, welches umfasst:
a) den Esterase-kodierenden Teil einer DNA-Sequenz, die in ein in *Escherichia coli* DSM 13977 vorhandenem Plasmid kloniert ist,
b) die als Nukleotide 629-911 und 971-2208 der SEQ ID NO:1 gezeigte Nukleinsäuresequenz,
c) ein Analog der in a) oder b) definierten Sequenz, welche für eine Esterase kodiert, welche zur stereoselektiven Hydrolyse chiraler Ester befähigt ist und eine Arylesteraseaktivität und eine Feruloylesteraseaktivität hat und
i) mindestens 60% Identität mit besagtem DNA-Sequenz aufweist, oder
ii) bei 60°C, 2xSSC, 0,5% SDS mit einem komplementären Strang der besagten DNA-Sequenz oder einer Untersequenz davon mit mindestens 100 Nukleotiden hybridisiert,
iii) eine allelische Variante davon ist, oder
d) ein komplementärer Strang von a), b) oder c).

5. Nukleinsäurekonstrukt umfassend das Polynukleotid nach Anspruch 3 oder 4, funktionsfähig verbunden mit einer oder mehr Kontrollsequenzen, die befähigt sind, die Expression der Esterase in einem geeigneten Expressionswirt zu steuern.

6. Rekombinanter Expressionsvektor umfassend das Nukleinsäurekonstrukt nach Anspruch 5, einen Promotor und transkriptionelle und translationelle Stoppsignale.

7. Rekombinante Wirtszelle, die mit dem Nukleinsäurekonstrukt nach Anspruch 6 transformiert ist.

8. Verfahren zum Herstellen einer Esterase, umfassend das Kultivieren der Wirtszelle nach Anspruch 7 unter Bedingungen, die für die Herstellung der Esterase förderlich sind, und das Gewinnen der Esterase.

9. Das Verfahren des vorherigen Anspruchs, wobei die Esterase von einem reifen Peptid der SEQ ID NO: 2 abgeleitet sein kann oder ein Analog davon ist, und die Wirtszelle ein transformierter Stamm von *A. oryzae* ist.

10. Verfahren zur stereoselektiven Hydrolyse eines chiralen Esters, umfassend Behandeln des Esters mit der Esterase nach Anspruch 1 oder 2.

11. Verfahren zum Herstellen eines optisch aktiven Carbonsäureesters, umfassend stereoselektives Hydrolysieren einer razemischen Mischung des Esters durch Behandeln mit der Esterase nach Anspruch 1 oder 2, und Gewinnen des optisch aktiven Esters.

12. Verfahren zum Herstellen einer optisch aktiven Carbonsäure, umfassend stereoselektives Hydrolysieren einer razemischen Mischung eines Esters der Säure durch Behandeln mit der Esterase nach Anspruch 1 oder 2, und Gewinnen der optisch aktiven Säure.

13. Verfahren zum Hydrolysieren eines Feruloylesters in Ferulinsäure und einen Alkohol, umfassend das Behandeln des Esters mit der Esterase nach Anspruch 1 oder 2.

## Revendications

1. Estérase qui est capable d'hydrolyser stéréosélectivement des esters chiraux et qui a une activité arylestérase et une activité féruloyl estérase et qui est :
a) un polypeptide codé par une partie codant une estérase de la séquence d'ADN clonée dans un plasmide présent dans *Escherichia coli* numéro de dépôt DSM 13977 ;
b) un polypeptide ayant comme peptide mature une séquence d'acides aminés montrée dans SEQ ID NO :2 ;
c) un analogue du polypeptide défini en (a) ou (b) qui:
i) a au moins 50 % d'identité avec ledit polypeptide,
ii) est immunologiquement réactif avec un anticorps dirigé contre ledit polypeptide sous forme purifiée, ou
iii) est un variant allélique dudit polypeptide ; ou
d) un polypeptide qui est codé par une séquence d'acide nucléique qui s'hybride à 60°C, 2 x SSC, 0,5 % de SDS avec un brin complémentaire de la séquence d'acide nucléique montrée comme étant les nucléotides 629-911 et 971-2208 de SEQ ID NO:1 ou une sous-séquence de celle-ci ayant au moins 100 nucléotides.

2. Estérase selon la revendication 1 qui a été isolée à partir d'une souche de *Aspergillus,* de préférence *A. oryzae.*

3. Polynucléotide comprenant une séquence d'acide nucléique qui code l'estérase selon la revendication 1 ou 2.

4. Polynucléotide qui comprend :
a) la partie codant une estérase de la séquence d'ADN clonée dans un plasmide présent dans *Escherichia coli* DSM 13977,
b) la séquence d'acide nucléique montrée comme étant les nucléotides 629-911 et 971-2208 de SEQ ID NO: 1,
c) un analogue de la séquence définie en a) ou b) qui code une estérase qui est capable d'hydrolyser stéréosélectivement des esters chiraux et qui a une activité arylestérase et une activité féruloyl estérase et
i) a au moins 60 % d'identité avec ledit séquence d'ADN, ou
ii) qui s'hybride à 60°C, 2 x SSC, 0,5 % de SDS avec un brin complémentaire de ladite séquence d'ADN ou d'une sous-séquence de celle-ci ayant au moins 100 nucléotides,
iii) est un variant allélique de celle-ci, ou
d) un brin complémentaire de a), b) ou c).

5. Construction d'acide nucléique comprenant le polynucléotide selon la revendication 3 ou 4 lié de manière opérationnelle à une ou plusieurs séquences de contrôle capables de diriger l'expression de l'estérase dans un hôte d'expression approprié.

6. Vecteur d'expression recombiné comprenant la construction d'acide nucléique selon la revendication 5, un promoteur et des signaux d'arrêt de la transcription et de la traduction.

7. Cellule hôte recombinée transformée avec la construction d'acide nucléique selon la revendication 6.

8. Procédé pour produire une estérase comprenant la culture de la cellule hôte selon la revendication 7 dans des conditions conduisant à la production de l'estérase, et la récupération de l'estérase.

9. Procédé selon la revendication précédente où l'estérase peut provenir du peptide mature de SEQ ID NO :2 ou est un analogue de celui-ci, et la cellule hôte est une souche transformée de *A. oryzae.*

10. Procédé pour l'hydrolyse stéréosélective d'un ester chiral, comprenant le traitement de l'ester avec l'estérase selon la revendication 1 ou 2.

11. Procédé de production d'un ester carboxylique optiquement actif, comprenant l'hydrolyse stéréosélective d'un mélange racémique de l'ester par traitement avec l'estérase selon la revendication 1 ou 2, et la récupération de l'ester optiquement actif.

12. Procédé de production d'un acide carboxylique optiquement actif, comprenant l'hydrolyse stéréosélective d'un mélange racémique d'un ester de l'acide par traitement avec l'estérase selon la revendication 1 ou 2, et la récupération de l'acide optiquement actif.

13. Procédé d'hydrolyse d'un féruloyl ester en acide férulique et un alcool, comprenant le traitement de l'ester avec l'estérase selon la revendication 1 ou 2.


### SEQUENCE LISTING

<110> Novozymes A/S
<120> Esterase
<130> 10141
<160> 27
<170> PatentIn version 3.0
<210> 1
   <211> 2346
   <212> DNA
   <213> Aspergillus oryzae
<220>
   <221> CDS
   <222> (572)..(911)
<220>
   <221> sig_peptide
   <222> (572)..(628)
<220>
   <221> misc_feature
   <222> (31)..()
   <223> n is unknown
<220>
   <221> CDS
   <222> (971)..(2208)
<220>
   <221> mat_peptide
   <222> (629) .. ()
<400> 1
   ttacttcacc aggatttagg gtcgagttcc ntcggtgccg aaaagaatgc ccgagcaatg     60
   tatttatgtg gccccaggac agtttaattg ccgatatcca agcctttcag gtgagtaaat    120
   tgcagagcgt gtgacaaggg taaccaggag aatactccgc attttgtggg gaaccccatg    180
   ggacgatctt tgggatgtgg agacactcat ttgaaaatga cagtgacttg tccagtcagc    240
   gctgctgaaa attgtctccc taatcccggc ttttccttgt cgaaaatgat tggggagtgc    300
   gtcacgtcac ggccaagctt tcctgcttag gaatttccta agctaataca tggtaccttc    360
   ctccggtcaa acttcggaga agccctagat aagggcacgg gatatagtcc gatcttcatg    420
   taccgacgga ttgaaagttt gaaacgctaa atgacatgtt ccttagtact gtcagcagtc    480
   tccggtatct ccgaggcagc tacatatata aagtcaccaa gctcacggca gaggaaaatg    540
   tctccgtgaa caacaaccac acccagccag t atg cct tca ctt cgc cgg ctt       592
                                      Met Pro Ser Leu Arg Arg Leu
                                                      -15
   ctg cct ttt ctt gct gca ggc tcc gcc gct ctg gca agc caa gat acg      640
   Leu Pro Phe Leu Ala Ala Gly Ser Ala Ala Leu Ala Ser Gln Asp Thr
           -10                 -5                  -1   1
   ttt caa ggc aag tgt act ggt ttt gca gac aag ata aac ctg cct aat      688
   Phe Gln Gly Lys Cys Thr Gly Phe Ala Asp Lys Ile Asn Leu Pro Asn
   5                   10                  15                  20
   gtg cgg gta aat ttt gtc aat tac gtg cct gga ggc acc aat ctt tct      736
   Val Arg Val Asn Phe Val Asn Tyr Val Pro Gly Gly Thr Asn Leu Ser
                   25                  30                  35
   ttg cca gat aat ccc acc agc tgc ggc aca acc tct caa gta gtg tcc      784
   Leu Pro Asp Asn Pro Thr Ser Cys Gly Thr Thr Ser Gln Val Val Ser
               40                  45                  50
   gag gat gtc tgc cgt att gcc atg gct gtt gca acc tca aac agt agc      832
   Glu Asp Val Cys Arg Ile Ala Met Ala Val Ala Thr Ser Asn Ser Ser
           55                  60                  65
   gaa atc acc ctt gaa gca tgg ctc cca caa aac tac act ggt cgt ttc      880
   Glu Ile Thr Leu Glu Ala Trp Leu Pro Gln Asn Tyr Thr Gly Arg Phe
       70                  75                  80
   ctg agt acg ggc aac ggt ggt ctc tca ggc t gtatgttcta cccggcaccg      931
   Leu Ser Thr Gly Asn Gly Gly Leu Ser Gly
   85                  90
   cgatgcgaca tggcacaact tcaaactaac gtcttacag gt att cag tac tat        984
                                              Cys Ile Gln Tyr Tyr
                                              95
   gat cta gcg tac acc tcc ggc ctc ggg ttt gcc acg gtt ggc gcc aac     1032
   Asp Leu Ala Tyr Thr Ser Gly Leu Gly Phe Ala Thr Val Gly Ala Asn
   100                 105                 110                 115
   agc ggc cat aac gga aca tcc ggg gag cct ttc tac cac cac cca gag     1080
   Ser Gly His Asn Gly Thr Ser Gly Glu Pro Phe Tyr His His Pro Glu
                   120                 125                 130
   gtc ctc gaa gac ttt gta cat cgt tca gtc cac act ggt gtc gtg gtt     1128
   Val Leu Glu Asp Phe Val His Arg Ser Val His Thr Gly Val Val Val
               135                 140                 145
   gga aag caa ttg aca aag ctt ttc tac gag gaa ggg ttc aag aag tcg     1176
   Gly Lys Gln Leu Thr Lys Leu Phe Tyr Glu Glu Gly Phe Lys Lys Ser
           150                 155                 160
   tac tac ctt ggt tgc tcc act ggt ggt cgg cag ggc ttt aaa tcc gtc     1224
   Tyr Tyr Leu Gly Cys Ser Thr Gly Gly Arg Gln Gly Phe Lys Ser Val
       165                 170                 175
   cag aaa tat ccc aat gac ttt gat ggt gtt gta gcc ggt gca ccg gca     1272
   Gln Lys Tyr Pro Asn Asp Phe Asp Gly Val Val Ala Gly Ala Pro Ala
   180                 185                 190                 195
   ttc aat atg atc aac ctc atg tca tgg agt gcc cac ttc tat tca atc     1320
   Phe Asn Met Ile Asn Leu Met Ser Trp Ser Ala His Phe Tyr Ser Ile
                   200                 205                 210
   acg ggg cca gtt ggg tcc gac aca tac cta tcc cct gac ctg tgg aat     1368
   Thr Gly Pro Val Gly Ser Asp Thr Tyr Leu Ser Pro Asp Leu Trp Asn
               215                 220                 225
   atc acc cat aag gag atc ctg cgt caa tgc gac ggt atc gat gga gca     1416
   Ile Thr His Lys Glu Ile Leu Arg Gln Cys Asp Gly Ile Asp Gly Ala
           230                 235                 240
   gag gac ggc att att gaa gac cca agt ctt tgc agc ccg gtt ctt gaa     1464
   Glu Asp Gly Ile Ile Glu Asp Pro Ser Leu Cys Ser Pro Val Leu Glu
       245                 250                 255
   gcg atc atc tgc aag cct ggt caa aac act acc gag tgt tta act ggc     1512
   Ala Ile Ile Cys Lys Pro Gly Gln Asn Thr Thr Glu Cys Leu Thr Gly
   260                 265                 270                 275
   aag caa gcc cat acc gtt cgc gaa att ttc tcc ccg ctg tac gga gtg     1560
   Lys Gln Ala His Thr Val Arg Glu Ile Phe Ser Pro Leu Tyr Gly Val
                   280                 285                 290
   aac ggc acc ttg ctt tat ccc cgc atg cag cct ggc tct gag gtg atg     1608
   Asn Gly Thr Leu Leu Tyr Pro Arg Met Gln Pro Gly Ser Glu Val Met
               295                 300                 305
   gct tct tcc ata atg tac aac ggc cag cct ttc cag tat agc gca gac     1656
   Ala Ser Ser Ile Met Tyr Asn Gly Gln Pro Phe Gln Tyr Ser Ala Asp
           310                 315                 320
   tgg tac cgc tat gtt gtc tac gag aac ccc aac tgg gat gca acc aag     1704
   Trp Tyr Arg Tyr Val Val Tyr Glu Asn Pro Asn Trp Asp Ala Thr Lys
       325                 330                 335
   ttc tcc gtc cgt gac gca gcc gtc gct ttg aag cag aac cca ttc aat     1752
   Phe Ser Val Arg Asp Ala Ala Val Ala Leu Lys Gln Asn Pro Phe Asn
   340                 345                 350                 355
   ctc cag acc tgg gac gca gat atc tcc tct ttc cgc aag gca ggc ggt     1800
   Leu Gln Thr Trp Asp Ala Asp Ile Ser Ser Phe Arg Lys Ala Gly Gly
                   360                 365                 370
   aaa gtc ctc acc tac cac ggt ctc atg gat caa ctt atc agc tcg gag     1848
   Lys Val Leu Thr Tyr His Gly Leu Met Asp Gln Leu Ile Ser Ser Glu
               375                 380                 385
   aac tcc aag ctt tac tat gcg cgc gtt gcg gaa acc atg aac gtc cct     1896
   Asn Ser Lys Leu Tyr Tyr Ala Arg Val Ala Glu Thr Met Asn Val Pro
       390                 395                 400
   ccg gaa gag ctg gac gag ttc tac cgc ttc ttt cag atc agt gga atg      1944
   Pro Glu Glu Leu Asp Glu Phe Tyr Arg Phe Phe Gln Ile Ser Gly Met
       405                 410                 415
   gcc cat tgc agt gga ggt gac gga gcg tac ggc att gga aac cag ctc      1992
   Ala His Cys Ser Gly Gly Asp Gly Ala Tyr Gly Ile Gly Asn Gln Leu
   420                 425                 430                 435
   gtg acc tat aac gat gcc aat cct gaa aac aac gtc ctc atg gct atg      2040
   Val Thr Tyr Asn Asp Ala Asn Pro Glu Asn Asn Val Leu Met Ala Met
                   440                 445                 450
   gtt cag tgg gtg gag aag ggc atc gcc ccg gag acc att cgt ggt gct      2088
   Val Gln Trp Val Glu Lys Gly Ile Ala Pro Glu Thr Ile Arg Gly Ala
               455                 460                 465
   aag ttt acc aat ggc acg ggc tcg gcc gtg gag tat act cgc aag cac      2136
   Lys Phe Thr Asn Gly Thr Gly Ser Ala Val Glu Tyr Thr Arg Lys His
           470                 475                 480
   tgc cgc tac cct cgc agg aat gta tac aag ggg cca ggg aac tac act      2184
   Cys Arg Tyr Pro Arg Arg Asn Val Tyr Lys Gly Pro Gly Asn Tyr Thr
       485                 490                 495
   gat gag aat gcc tgg caa tgt gtt taaattgttg aagtattgta catatatttg     2238
   Asp Glu Asn Ala Trp Gln Cys Val
   500                 505
   ctcatagagg caagacgttt gcatgtcttg ataattattt attcgcccat catagcagat    2298
   agaatataag accacgtcct acgaaactcg cagtgcactt gtataatt                 2346
<210> 2
   <211> 526
   <212> PRT
   <213> Aspergillus oryzae
<220>
   <221> misc_feature
   <222> (31)..()
   <223> n is unknown
<400> 2
   Met Pro Ser Leu Arg Arg Leu Leu Pro Phe Leu Ala Ala Gly Ser Ala
                   -15                 -10                 -5
   Ala Leu Ala Ser Gln Asp Thr Phe Gln Gly Lys Cys Thr Gly Phe Ala
           -1  1               5                   10
   Asp Lys Ile Asn Leu Pro Asn Val Arg Val Asn Phe Val Asn Tyr Val
       15                  20                  25
   Pro Gly Gly Thr Asn Leu Ser Leu Pro Asp Asn Pro Thr Ser Cys Gly
   30                  35                  40                  45
   Thr Thr Ser Gln Val Val Ser Glu Asp Val Cys Arg Ile Ala Met Ala
                   50                  55                  60
   Val Ala Thr Ser Asn Ser Ser Glu Ile Thr Leu Glu Ala Trp Leu Pro
               65                  70                  75
   Gln Asn Tyr Thr Gly Arg Phe Leu Ser Thr Gly Asn Gly Gly Leu Ser
           80                  85                  90
   Gly Cys Ile Gln Tyr Tyr Asp Leu Ala Tyr Thr Ser Gly Leu Gly Phe
       95                  100                 105
   Ala Thr Val Gly Ala Asn Ser Gly His Asn Gly Thr Ser Gly Glu Pro
   110                 115                 120                 125
   Phe Tyr His His Pro Glu Val Leu Glu Asp Phe Val His Arg Ser Val
                   130                 135                 140
   His Thr Gly Val Val Val Gly Lys Gln Leu Thr Lys Leu Phe Tyr Glu
               145                 150                 155
   Glu Gly Phe Lys Lys Ser Tyr Tyr Leu Gly Cys Ser Thr Gly Gly Arg
           160                 165                 170
   Gln Gly Phe Lys Ser Val Gln Lys Tyr Pro Asn Asp Phe Asp Gly Val
       175                 180                 185
   Val Ala Gly Ala Pro Ala Phe Asn Met Ile Asn Leu Met Ser Trp Ser
   190                 195                 200                 205
   Ala His Phe Tyr Ser Ile Thr Gly Pro Val Gly Ser Asp Thr Tyr Leu
                   210                 215                 220
   Ser Pro Asp Leu Trp Asn Ile Thr His Lys Glu Ile Leu Arg Gln Cys
               225                 230                 235
   Asp Gly Ile Asp Gly Ala Glu Asp Gly Ile Ile Glu Asp Pro Ser Leu
           240                 245                 250
   Cys Ser Pro Val Leu Glu Ala Ile Ile Cys Lys Pro Gly Gln Asn Thr
       255                 260                 265
   Thr Glu Cys Leu Thr Gly Lys Gln Ala His Thr Val Arg Glu Ile Phe
   270                 275                 280                 285
   Ser Pro Leu Tyr Gly Val Asn Gly Thr Leu Leu Tyr Pro Arg Met Gln
                   290                 295                 300
   Pro Gly Ser Glu Val Met Ala Ser Ser Ile Met Tyr Asn Gly Gln Pro
   305 310 315
   Phe Gln Tyr Ser Ala Asp Trp Tyr Arg Tyr Val Val Tyr Glu Asn Pro
   320 325 330
   Asn Trp Asp Ala Thr Lys Phe Ser Val Arg Asp Ala Ala Val Ala Leu
   335 340 345
   Lys Gln Asn Pro Phe Asn Leu Gln Thr Trp Asp Ala Asp Ile Ser Ser
   350 355 360 365
   Phe Arg Lys Ala Gly Gly Lys Val Leu Thr Tyr His Gly Leu Met Asp
   370 375 380
   Gln Leu Ile Ser Ser Glu Asn Ser Lys Leu Tyr Tyr Ala Arg Val Ala
   385 390 395
   Glu Thr Met Asn Val Pro Pro Glu Glu Leu Asp Glu Phe Tyr Arg Phe
   400 405 410
   Phe Gln Ile Ser Gly Met Ala His Cys Ser Gly Gly Asp Gly Ala Tyr
   415 420 425
   Gly Ile Gly Asn Gln Leu Val Thr Tyr Asn Asp Ala Asn Pro Glu Asn
   430 435 440 445
   Asn Val Leu Met Ala Met Val Gln Trp Val Glu Lys Gly Ile Ala Pro
   450 455 460
   Glu Thr Ile Arg Gly Ala Lys Phe Thr Asn Gly Thr Gly Ser Ala Val
   465 470 475
   Glu Tyr Thr Arg Lys His Cys Arg Tyr Pro Arg Arg Asn Val Tyr Lys
   480 485 490
   Gly Pro Gly Asn Tyr Thr Asp Glu Asn Ala Trp Gln Cys Val
   495 500 505
<210> 3
   <211> 18
   <212> PRT
   <213> Aspergillus oryzae
<220>
   <221> misc_feature
   <222> 0..0
   <223> 161299Afr15
<400> 3
   Val Leu Thr Tyr His Gly Leu Met Asp Gln Leu Ile Ser Ser Glu Asn
   1 5 10 15
   Ser Lys
<210> 4
   <211> 18
   <212> PRT
   <213> Aspergillus oryzae
<220>
   <221> misc_feature
   <222> ()..()
   <223> 161299Afr17
<400> 4
   Gln Asn Pro Phe Asn Leu Gln Thr Trp Asp Ala Asp Ile Ser Ser Phe
   1 5 10 15
   Arg Lys
<210> 5
   <211> 21
   <212> PRT
   <213> Aspergillus oryzae
<220>
   <221> misc_feature
   <222> () .. ()
   <223> 161299Afr23
<400> 5
   Tyr Pro Asn Asp Phe Asp Gly Val Val Ala Gly Ala Pro Ala Phe Asn
   1 5 10 15
   Met Ile Asn Leu Met
   20
<210> 6
   <211> 27
   <212> DNA
   <213> Artificial/unknown
<220>
   <221> misc_feature
   <222> () .. ()
   <223> B2716F09
<400> 6
   cccgccttca acatgatcaa cctsatg 27
<210> 7
   <211> 26
   <212> DNA
   <213> Artificial/unknown
<220>
   <221> misc_feature
   <222> ()..()
   <223> B2716F11
<400> 7
   atsagctggt ccatsagrcc gtgrta 26
<210> 8
   <211> 24
   <212> DNA
   <213> Artificial/unknown
<220>
   <221> misc_feature
   <222> ()..()
   <223> -48 reverse
<400> 8
   agcggataac aatttcacac agga 24
<210> 9
   <211> 17
   <212> DNA
   <213> Artificial/unknown
<220>
   <221> misc_feature
   <222> () .. ()
   <223> -40 universal
<400> 9
   gttttcccag tcacgac 17
<210> 10
   <211> 22
   <212> DNA
   <213> Artificial/unknown
<220>
   <221> misc_feature
   <222> ()..()
   <223> B2998F06
<400> 10
   ccccgtgatt gaatagaagt gg 22
<210> 11
   <211> 22
   <212> DNA
   <213> Artificial/unknown
<220>
   <221> misc_feature
   <222> ()..()
   <223> B3591G12
<400> 11
   tccataatgt acaacggcca gc 22
<210> 12
   <211> 22
   <212> DNA
   <213> Artificial/Unknown
<220>
   <221> misc_feature
   <222> ()..()
   <223> B2998F07
<400> 12
   gacgcagata tctcctcttt cc 22
<210> 13
   <211> 21
   <212> DNA
   <213> Artificial/unknown
<220>
   <221> misc_feature
   <222> ()··()
   <223> B3864E07
<400> 13
   gattggtgcc tccaggcacg t 21
<210> 14
   <211> 21
   <212> DNA
   <213> Artificial/unknown
<220>
   <221> misc_feature
   <222> ().. ()
   <223> 83864E08
<400> 14
   gcaagtgtac tggttttgca g 21
<210> 15
   <211> 21
   <212> DNA
   <213> Artificial/unknown
<220>
   <221> misc_feature
   <222> ()..()
   <223> B3998D09
<400> 15
   ttatcaagac atgcaaacgt c 21
<210> 16
   <211> 20
   <212> DNA
   <213> Artificial/unknown
<220>
   <221> misc_feature
   <222> ()..()
   <223> 83998D10
<400> 16
   cttggttgct ccactggtgg 20
<210> 17
   <211> 20
   <212> DNA
   <213> Artificial/unknown
<220>
   <221> misc_feature
   <222> ()..()
   <223> 83998D11
<400> 17
   tccagctctt ccggagggac 20
<210> 18
   <211> 22
   <212> DNA
   <213> Artificial/unknown
<220>
   <221> misc_feature
   <222> () .. ()
   <223> B3591G11
<400> 18
   aaagcaaggt gccgttcact cc 22
<210> 19
   <211> 11
   <212> DNA
   <213> Artificial/unknown
<220>
   <221> misc_feature
   <222> ()..()
   <223> Pna2/tpi nucleotide 134-144
<400> 19
   gtactaaaac c 11
<210> 20
   <211> 11
   <212> DNA
   <213> Artificial/unknown
<220>
   <221> misc_feature
   <222> ()..()
   <223> Pna2/tpi nucleotide 134-144 altered
<400> 20
   ccgttaaatt t 11
<210> 21
   <211> 45
   <212> DNA
   <213> Artificial/unknown
<220>
   <221> misc_feature
   <222> ()..()
   <223> 141223
<400> 21
   ggatgctgtt gactccggaa atttaacggt ttggtcttgc atccc 45
<210> 22
   <211> 14
   <212> DNA
   <213> Artificial/unknown
<220>
   <221> misc_feature
   <222> ()..()
   <223> Pna2/tpi 423-436
<400> 22
   atgcaattta aact 14
<210> 23
   <211> 14
   <212> DNA
   <213> Artificial/unknown
<220>
   <221> misc_feature
   <222> ()..()
   <223> Pna2/tpi nucleotide 423-436 altered
<400> 23
   cggcaattta acgg 14
<210> 24
   <211> 44
   <212> DNA
   <213> Artificial/unknown
<220>
   <221> misc_feature
   <222> ()..()
   <223> 141222
<400> 24
   ggtattgtcc tgcagacggc aatttaacgg cttctgcgaa tcgc 44
<210> 25
   <211> 34
   <212> DNA
   <213> Artificial/unknown
<220>
   <221> misc_feature
   <222> ()..()
   <223> B6093H05
<400> 25
   ttggatcctt caccatgcct tcacttcgcc ggct 34
<210> 26
   <211> 30
   <212> DNA
   <213> Artificial/unknown
<220>
   <221> misc_feature
   <222> ()..()
   <223> B6093H03
<400> 26
   atctcgagtt taaacacatt gccaggcatt 30
<210> 27
   <211> 572
   <212> DNA
   <213> Aspergillus oryzae
<220>
   <221> misc_feature
   <222> ()..()
   <223> PCR fragment
<400> 27
   cccgccttca acatgatcaa cctgatgtca tggagtgccc acttctattc aatcacgggg     60
   ccagttgggt ccgacacata cctatcccct gacctgtgga atatcaccca taaggagatc    120
   ctgcgtcaat gcgacggtat cgatggagca gaggacggca ttattgaaga cccaagtctt    180
   tgcagcccgg ttcttgaagc gatcatctgc aagcctggtc aaaacactac cgagtgttta    240
   actggcaagc aagcccatac cgttcgcgaa attttctccc cgctgtacgg agtgaacggc    300
   accttgcttt atccccgcat gcagcctggc tctgaggtga tggcttcttc cataatgtac    360
   aacggccagc ctttccagta tagcgcagac tggtaccgct atgttgtcta cgagaacccc    420
   aactgggatg caaccaagtt ctccgtccgt gacgcagccg tcgctttgaa gcagaaccca    480
   ttcaayctcc agacctggga cgcagatatc tcctctttcc gcaaggcagg cggtaaagtc    540
   ctcacctatc acggcctgat ggaccagctg at                                  572